# EUROPÄISCHE PATENTANMELDUNG

(11) **EP 4 342 500 A2**
(43) Veröffentlichungstag der Anmeldung: **27.03.2024**
(21) Anmeldenummer: 23195537.8
(22) Anmeldetag: 05.09.2023
(51) Int. Cl.: A61L 2/10

(54) **VORRICHTUNG UND VERFAHREN ZUM DESINFIZIEREN VON BEHÄLTERN**

(30) Priorität: 05.09.2022 DE 102022122422
(71) Anmelder: KRONES AG, 93073 Neutraubling (DE)
(72) Erfinder: Schumacher, Andreas, 93073 Neutraubling (DE)
(74) Vertreter: Nordmeyer, Philipp Werner

(57) **Zusammenfassung**

Vorrichtung (1) und Verfahren zum Desinfizieren eines Behälters (2) mit einer Behältermündung (2a), vorzugsweise in einer Getränkeabfüllanlage, wobei die Vorrichtung (1) aufweist: eine Transportvorrichtung, die eingerichtet ist, um den Behälter (2) entlang eines Förderwegs (F) zu transportieren; eine Strahlungseinrichtung (40) mit zumindest einer Strahlungssonde (41), die eingerichtet ist, um eine desinfizierende Strahlung, vorzugsweise UV-Strahlung, zu emittieren, wobei die Strahlungssonde (41) und/oder die Transportvorrichtung so relativ zueinander bewegbar sind, dass die Strahlungssonde (41) durch die Behältermündung (2a) zumindest teilweise in den Behälter (2) einbringbar ist; eine Steuerung (60), die mit der Transportvorrichtung und der Strahlungseinrichtung (40) in Kommunikation steht und eingerichtet ist, um diese für eine Strahlungsbehandlung zu veranlassen, die Strahlungssonde (41) durch die Behältermündung (2a) in den Behälter (2) einzubringen und mit der desinfizierenden Strahlung zu behandeln.

## Beschreibung

### Technisches Gebiet

Die vorliegende Erfindung betrifft eine Vorrichtung und ein Verfahren zum Desinfizieren eines Behälters, insbesondere in einer Getränkeabfüllanlage.

### Stand der Technik

Auf dem Gebiet der Getränkeabfüllanlagen ist es bekannt, die zu befüllenden Behälter vor dem eigentlichen Befüllen mit einem Füllprodukt zu entkeimen, üblicherweise unter Anwendung wässriger oder gasförmiger Desinfektionsmittel wie beispielsweise Wasserstoffperoxyd oder Peressigsäure. Durch die Sterilisierung beziehungsweise Desinfizierung wird der Eintrag von schädlichen Mikroorganismen und/oder Sporen in das Füllprodukt vermieden oder zumindest deutlich vermindert, wodurch die Lagerdauer des abgefüllten Getränkes erhöht und die Produkteigenschaften des Füllprodukts nach der Befüllung stabilisiert werden. Zu diesem Zweck wird das desinfizierende Fluid mittels Düsen auf Oberflächen, insbesondere Innenflächen des Behälters appliziert und nach einer definierten Einwirkzeit wieder ausgespült, um eine Restkonzentrationen im Behälter so gering wie möglich zu halten.

Ein Verfahren zur Desinfektion von Behältern vor dem Befüllen mittels gasförmigen Wasserstoffperoxyds ist beispielsweise in der EP 2 008 667 A1 beschrieben.

Für eine auf Chemikalien basierende Behälterentkeimung muss sowohl in technischer Hinsicht (Absaugung, Gewährleistung hermetischer Systeme, Konzentrations-, Durchfluss- und Temperaturmessung usw.) als auch medientechnisch (Wasser, Chemikalien usw.) ein hoher Aufwand betrieben werden. Dies wirkt sich nachteilig auf die Verwendung von Ressourcen, Wartungsaufwand, Kosten und dergleichen aus.

Alternative Desinfektions- beziehungsweise Sterilisationsverfahren beruhen auf der Anwendung von Strahlung. So ist die Sterilisierung von Behältern mittels Elektronenstrahlen etwa aus der EP 2 688 073 A2 bekannt. Die DE 10 2013 013 590 A1 schlägt die Anwendung von UV-Strahlung vor.

Eine Schwierigkeit bei der Anwendung von Strahlung jedweder Art besteht in einer möglichen Abschattung von zu desinfizierenden Flächen, d.h. Flächen, die von der Strahlungsquelle nicht oder nicht direkt erreichbar sind. Dieses Problem tritt insbesondere bei komplizierten Behältergeometrien auf, deren Wölbungen, Vorsprünge, Hinterschnitte usw. den Strahlungsweg blockieren können. Dies kann dazu führen, dass die Zuverlässigkeit von strahlungsbasierten Desinfektionsverfahren vermindert ist.

### Darstellung der Erfindung

Eine Aufgabe der vorliegenden Erfindung besteht darin, die Desinfektion von Behältern zu verbessern, insbesondere die Zuverlässigkeit und/oder Gründlichkeit bei der Anwendung von desinfizierender Strahlung zu erhöhen.

Die Aufgabe wird durch eine Vorrichtung mit den Merkmalen des Anspruchs 1 sowie ein Verfahren mit den Merkmalen des nebengeordneten Verfahrensanspruchs gelöst. Vorteilhafte Weiterbildungen folgen aus den Unteransprüchen, der folgenden Darstellung der Erfindung sowie der Beschreibung bevorzugter Ausführungsbeispiele.

Die Vorrichtung gemäß der Erfindung ist zum Desinfizieren eines oder mehrerer Behälter konzipiert. Die Vorrichtung kommt besonders bevorzugt in einer Getränkeabfüllanlage zur Anwendung, beispielsweise zum Abfüllen von Wasser (still oder karbonisiert), Softdrinks, Bier, Wein, Mischgetränken, Milchprodukten und dergleichen. Durch die Desinfizierung des Behälters wird der Eintrag von schädlichen Mikroorganismen, Sporen usw. in das Füllprodukt vermieden oder zumindest deutlich vermindert, was insbesondere im Bereich der Lebensmittelverarbeitung von großer Bedeutung ist.

Der Begriff "Desinfizieren" umfasst hierin eine Sterilisation, bei der im Wesentlichen eine vollständige Entkeimung angestrebt wird.

Die Vorrichtung gemäß der Erfindung umfasst eine Transportvorrichtung, die eingerichtet ist, um den Behälter entlang eines Förderwegs zu transportieren, und eine Strahlungseinrichtung mit zumindest einer Strahlungssonde, die eingerichtet ist, um eine desinfizierende Strahlung, vorzugsweise UV-Strahlung, zu emittieren. Die Strahlungssonde und/oder die Transportvorrichtung sind hierbei relativ zueinander bewegbar eingerichtet, so dass die Strahlungssonde durch die Behältermündung zumindest teilweise in den Behälter eintauchbar beziehungsweise einbringbar ist.

"Eintauchbar" beziehungsweise "einbringbar" bedeutet in diesem Zusammenhang, dass die Strahlungssonde durch die Ebene der Behältermündung zumindest teilweise in den Behälter eindringen kann. In anderen Worten, die Strahlungsquelle ist während der Strahlungsbehandlung nicht außerhalb des Behälters, sondern zumindest teilweise innerhalb des Behälters angeordnet.

Die Vorrichtung weist ferner eine Steuerung auf, die mit der Transportvorrichtung und der Strahlungseinrichtung in Kommunikation steht und eingerichtet ist, um diese für eine Strahlungsbehandlung zu veranlassen, die Strahlungssonde durch die Behältermündung in den Behälter einzutauchen beziehungsweise einzubringen und mit der desinfizierenden Strahlung zu behandeln. Die Strahlungsbehandlung findet hierbei besonders bevorzugt während des Transports des Behälters durch die Transportvorrichtung statt.

Indem die Strahlungssonde in den offenen und leeren, d.h. noch zu befüllenden, Behälter eintaucht, können Abschattungen und dadurch Behälterflächen, die durch eine etwaige Abschattung anderweitig nicht oder nur indirekt bestrahlt werden könnten, minimiert werden. Die Desinfektion erfolgt auf diese Weise besonders gründlich und zuverlässig.

Nach Abschluss der Strahlungsbehandlung und zumindest vor dem nächsten Behandlungsschritt und/oder vor der Übergabe der dann behandelten Behälter an eine nachfolgende Transportvorrichtung wird die Strahlungssonde wieder aus dem dann behandelten Behälter herausgezogen. Entsprechend kann der Behälter dann an nachfolgende Bearbeitungsstationen, beispielsweise an einen Füller zum Befüllen des behandelten Behälters mit einem Füllprodukt, übergeben werden und beispielsweise befüllt werden.

Die Steuervorrichtung ist bevorzugt dazu eingerichtet, nach Abschluss der Behandlung die Strahlungssonde durch die Behältermündung wieder aus dem Behälter herauszubewegen.

Vorzugsweise ist die Strahlungssonde verschiebbar, insbesondere vertikal verschiebbar, ausgebildet, wodurch die Relativbewegung zwischen Strahlungssonde und Behälter auf technisch einfache Weise realisiert ist. Alternativ oder zusätzlich kann der Behälter so verschiebbar gehalten werden, dass die Strahlungssonde in den Behälter eintauchen kann.

Es sei darauf hingewiesen, dass sich räumliche Angaben wie "vertikal", "unten", "oben" usw. auf die Schwerkraftrichtung beziehen und durch den bestimmungsgemäßen Gebrauch der Vorrichtung klar definiert sind.

Vorzugsweise umfasst die Transportvorrichtung ein um eine Drehachse drehbares Behandlungskarussell, an dessen Außenumfang mehrere Haltevorrichtungen installiert sind, die eingerichtet sind, um je einen Behälter zu halten und durch Drehung des Behandlungskarussells entlang des Förderwegs zu transportieren. Der Förderweg ist im Fall einer solchen Rundläuferbauweise eine kreisförmige Trajektorie beziehungsweise ein Teilkreis. Auf diese Weise kann ein kontinuierlicher Strom von Behältern behandelt werden. Die Behälter werden beispielsweise über einen Einlaufstern zugeführt und an das Behandlungskarussell übergeben, wobei die Behälter von den entsprechenden Haltevorrichtungen empfangen und während des Transports entlang des Förderwegs gehalten werden. Nach erfolgter Behandlung werden die Behälter beispielsweise über einen Auslaufstern abtransportiert.

Vorzugsweise weist die Vorrichtung mehrere Strahlungseinrichtungen auf, die am Außenumfang des Behandlungskarussells installiert und je einer Haltevorrichtung zugeordnet sind, so dass mehrere Behälter gleichzeitig behandelbar sind.

Vorzugsweise ist die Transportvorrichtung eingerichtet, um den Behälter mit der Behältermündung nach unten orientiert zu transportieren, insbesondere während der Strahlungsbehandlung. Dadurch können etwaige Verunreinigungen, Partikel usw. ungehindert herausfallen, wodurch die Behandlung weiter verbessert wird.

Zu diesem Zweck sind die Haltevorrichtungen vorzugsweise eingerichtet, um den entsprechenden, gehaltenen Behälter von einer aufrechten Lage, in der die Behältermündung nach oben orientiert ist, in eine aufgeschwenkte Lage, in der die Behältermündung nach unten orientiert ist, zu schwenken, und vice versa.

Vorzugsweise ist die Strahlungseinrichtung eingerichtet, um über die Strahlungssonde eine gepulste Strahlung, vorzugsweise mit einer Pulsdauer im Bereich von 0,2 ms bis 0,4 ms, besonders bevorzugt ca. 0,3 ms, zu emittieren, wodurch die Behandlung besonders gründlich durchführbar ist. Die Wellenlänge der Strahlung liegt vorzugsweise im Bereich von 300 nm bis 400 nm, besonders bevorzugt ca. 350 nm. Die gepulste UV-Strahlung kann beispielsweise durch eine Xenon-Lampe erzeugt werden.

Vorzugsweise weist die Vorrichtung ferner eine Vorbehandlungseinrichtung mit zumindest einer Behandlungsdüse auf, die eingerichtet ist, um den Behälter mit einem Behandlungsmedium auszuspülen. Die Steuerung steht in diesem Fall mit der Vorbehandlungseinrichtung in Kommunikation und ist eingerichtet, um diese für eine Vorbehandlung zu veranlassen, den Behälter vor der Strahlungsbehandlung durch die Strahlungseinrichtung mit dem Behandlungsmedium auszuspülen. Die Vorbehandlung reinigt den Behälter und bereitet diesen für die desinfizierende Strahlungsbehandlung vor, wodurch die Behandlung noch gründlicher erfolgen kann.

Das Behandlungsmedium ist vorzugsweise Sterilluft, insbesondere ionisierte Sterilluft. In diesem Fall wirkt das Behandlungsmedium im Wesentlichen rein mechanisch, indem etwaige Partikel usw. ausgespült beziehungsweise ausgeblasen werden. Zusätzlich oder alternativ kann das Behandlungsmedium auch eine chemische Wirkung entfalten, beispielsweise desinfizierend wirken, um die Gesamtbehandlung, umfassend die Vor- und Strahlungsbehandlung, zu optimieren.

Vorzugsweise ist die Behandlungsdüse durch die Behältermündung zumindest teilweise in den Behälter eintauchbar, insbesondere vertikal verschiebbar, wodurch Abschattungen und dadurch Behälterflächen, die durch eine etwaige Abschattung anderweitig nicht oder nur indirekt mit dem Behandlungsmedium beaufschlagt werden könnten, minimiert werden.

Die vorstehend beschriebene Vorrichtung ist für die Behandlung von Behältern mit einfacher Geometrie und/oder großer Behältermündung, insbesondere Dosen, besonders geeignet. Die Strahlungssonde und/oder die Behandlungsdüse können wegen der großen Behältermündung tief in den Behälter eintauchen. Ferner gewährleistet eine schlichte, zylindrische Behälterform, insbesondere die Form eines geraden Kreiszylinders, eine schattenfreie Behandlung.

Die oben genannte Aufgabe wird ferner durch ein Verfahren zum Desinfizieren eines Behälters mit einer Behältermündung, vorzugsweise in einer Getränkeabfüllanlage, gelöst, wobei das Verfahren aufweist: Transportieren des Behälters mittels einer Transportvorrichtung entlang eines Förderwegs, wobei der Behälter vorzugsweise mit der Behältermündung nach unten orientiert transportiert wird; Eintauchen einer Strahlungssonde einer Strahlungseinrichtung durch die Behältermündung in den Behälter, vorzugsweise während des Transports; und Durchführen einer Strahlungsbehandlung durch Emittieren einer desinfizierenden Strahlung, vorzugsweise UV-Strahlung, durch die Strahlungssonde, um den Behälter mit der desinfizierenden Strahlung zu behandeln. Die Strahlungsbehandlung erfolgt hierbei im eingetauchten Zustand der Strahlungssonde.

Nach Abschluss der Strahlungsbehandlung und vor der Übergabe an eine stromabwärts gelegene Transport- oder Behandlungsstation wird die Strahlungssonde wieder aus dem Behälter herausgeführt.

Die Merkmale, technischen Wirkungen, Vorteile sowie Ausführungsbeispiele, die in Bezug auf die Vorrichtung beschrieben wurden, gelten analog für das Verfahren.

So ist die desinfizierende Strahlung aus den oben genannten Gründen vorzugsweise eine gepulste Strahlung, insbesondere mit einer Pulsdauer im Bereich von 0,2 ms bis 0,4 ms. Vorzugsweise liegt die Wellenlänge der Strahlung im Bereich von 300 nm bis 400 nm.

Vorzugsweise wird aus den oben genannten Gründen vor der Strahlungsbehandlung eine Vorbehandlung durchgeführt, indem der Behälter mit einem Behandlungsmedium, insbesondere Sterilluft, ausgespült wird.

Vorzugsweise weist der Behälter aus den oben genannten Gründen im Wesentlichen die Form eines senkrechten Kreiszylinders auf. Besonders bevorzugt ist der Behälter eine Dose, beispielsweise aus Aluminium oder Weißblech.

Vorzugsweise umfasst die Transportvorrichtung ein um eine Drehachse drehbares Behandlungskarussell, an dessen Außenumfang mehrere Haltevorrichtungen installiert sind, die eingerichtet sind, um je einen Behälter zu halten und durch Drehung des Behandlungskarussells entlang eines kreisförmigen Förderwegs zu transportieren und zu schwenken, wobei das Behandlungskarussell mehrere Behandlungsbereiche durchfährt.

Die Behandlungsbereiche umfassen vorzugsweise einen ersten Behandlungsbereich, in dem der Behälter durch die entsprechende Haltevorrichtung von einer aufrechten Lage, in der die Behältermündung nach oben orientiert ist, in eine aufgeschwenkte Lage, in der die Behältermündung nach unten orientiert ist, geschwenkt wird; einen zweiten Behandlungsbereich, in dem die Vorbehandlung des Behälters mittels der Vorbehandlungseinrichtung durchgeführt wird; einen dritten Behandlungsbereich, in dem die Strahlungsbehandlung des Behälters mittels der Strahlungseinrichtung durchgeführt wird; und einen vierten Behandlungsbereich, in dem der Behälter durch die entsprechende Haltevorrichtung in die aufrechte Lage, in der die Behältermündung nach oben orientiert ist, zurückgeschwenkt wird.

Weitere Vorteile und Merkmale der vorliegenden Erfindung sind aus der folgenden Beschreibung bevorzugter Ausführungsbeispiele ersichtlich. Die darin beschriebenen Merkmale können alleinstehend oder in Kombination mit einem oder mehreren der oben dargelegten Merkmale umgesetzt werden, insofern sich die Merkmale nicht widersprechen. Die folgende Beschreibung bevorzugter Ausführungsbeispiele erfolgt dabei mit Bezug auf die begleitenden Zeichnungen.

### Kurze Beschreibung der Figuren

Bevorzugte weitere Ausführungsformen der Erfindung werden durch die nachfolgende Beschreibung der Figuren näher erläutert. Dabei zeigen:
- Figur 1: schematisch eine Vorrichtung zum Desinfizieren von Behältern in einer Draufsicht, umfassend eine Strahlungseinrichtung und eine Vorbehandlungseinrichtung;
- Figur 2: schematisch eine Strahlungseinrichtung in einem Behandlungsbetrieb gemäß einem Ausführungsbeispiel;
- Figur 3: schematisch eine Strahlungseinrichtung in einem Behandlungsbetrieb gemäß einem weiteren Ausführungsbeispiel;
- Figur 4: schematisch eine Vorbehandlungseinrichtung gemäß einem Ausführungsbeispiel, das sich von der Figur 1 unterscheidet; und
- Figur 5: schematisch ein Behandlungskarussell der Vorrichtung zum Desinfizieren von Behältern gemäß einem weiteren Ausführungsbeispiel.

### Detaillierte Beschreibung bevorzugter Ausführungsbeispiele

Im Folgenden werden bevorzugte Ausführungsbeispiele anhand der Figuren beschrieben. Dabei sind gleiche, ähnliche oder gleichwirkende Elemente in den Figuren mit identischen Bezugszeichen versehen, und auf eine wiederholte Beschreibung dieser Elemente wird teilweise verzichtet, um Redundanz zu vermeiden.

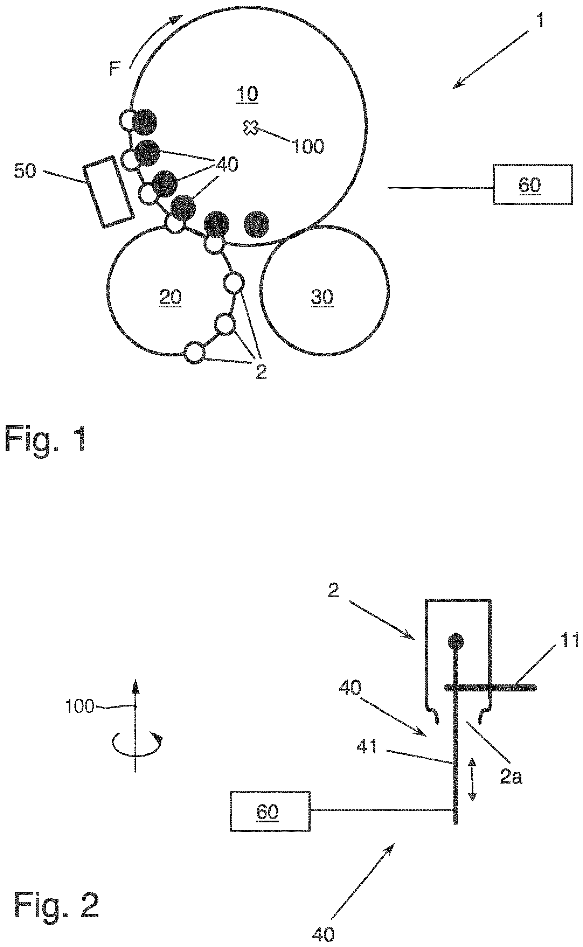

Die Figur 1 zeigt eine Vorrichtung 1 zum Desinfizieren von Behältern 2, insbesondere Dosen. Die Vorrichtung 1 kommt besonders bevorzugt in einer Getränkeabfüllanlage zur Anwendung, beispielsweise zum Abfüllen von Wasser (still oder karbonisiert), Softdrinks, Bier, Wein, Mischgetränken, Milchprodukten und dergleichen.

Die Vorrichtung 1 ist im vorliegenden Ausführungsbeispiel in Rundläuferbauweise ausgeführt, d.h. sie umfasst ein um eine Drehachse 100 drehbares Behandlungskarussell 10, an dessen Außenumfang die zu desinfizierenden Behälter 2 über geeignete Haltevorrichtungen 11 (vgl. Figuren 2, 3 und 4) gehalten und entlang eines Förderwegs F transportiert werden. Das Behandlungskarussell 10 fungiert als Transportvorrichtung für die Behälter 2. Der Förderweg F ist im Fall der Rundläuferbauweise eine kreisförmige Trajektorie beziehungsweise ein Teilkreis. Auf diese Weise kann ein kontinuierlicher Strom von Behältern 2 behandelt werden.

Die Haltevorrichtungen 11 können als Halteklammern, Haltetaschen, Greifeinrichtungen, bodenseitige Teller oder auf andere Weise ausgebildet sein und sorgen dafür, dass die Behälter 2 während des Transports und während der Behandlung die durch den Förderweg F vorgegebene Trajektorie in einem definierten Abstand abfahren. Besonders bevorzugt sind die Haltevorrichtungen 11 eingerichtet, um die Behälter 2 auf den Kopf zu schwenken oder auf dem Kopf stehend zu halten und zu transportieren (vgl. Figuren 2, 3 und 4).

Die Behälter 2 können alternativ durch eine geeignete Transportvorrichtung entlang eines linearen Förderwegs F oder entlang einer anderen Trajektorie transportiert werden, d.h. die Vorrichtung 1 ist nicht auf eine Rundläuferbauweise beschränkt.

Die Behälter 2 werden beispielsweise über einen Einlaufstern 20 zugeführt und an das Behandlungskarussell 10 übergeben, wobei die Behälter 2 von den Haltevorrichtungen 11 empfangen und während des Transports entlang des Förderwegs F gehalten werden. Nach erfolgter Behandlung werden die Behälter 2 beispielsweise über einen Auslaufstern 30 abtransportiert.

Die Vorrichtung 1 weist eine oder mehrere Strahlungseinrichtungen 40 auf, die eingerichtet sind, um die zu desinfizierenden Behälter 2 mit einer desinfizierenden Strahlung zu behandeln.

Hierbei handelt es sich vorzugsweise um eine gepulste Strahlung, insbesondere eine gepulste UV-Strahlung, beispielsweise mit einer Pulsdauer im Bereich von 0,2 ms bis 0,4 ms, vorzugsweise ca. 0,3 ms. Die Wellenlänge der Strahlung liegt vorzugsweise im Bereich von 300 nm bis 400 nm, sie beträgt insbesondere ca. 350 nm. Die UV-Strahlung kann beispielsweise durch eine Xenon-Lampe erzeugt werden.

Die Strahlungseinrichtung 40 kann außerhalb des Behandlungskarussells 10 angeordnet oder daran angebracht beziehungsweise Bestandteil desselben sein. Im letzteren Fall sind vorzugsweise mehrere Strahlungseinrichtungen 40 vorgesehen, die den Behältern 2 beziehungsweise deren Haltevorrichtungen 11 zugeordnet sind. Die Strahlungseinrichtungen 40 fungieren in diesem Fall als mitlaufende Behandlungsorgane, wodurch sich die Behandlungszeit im Vergleich zu einer relativ zu den transportierten Behältern 2 stationären Strahlungseinrichtung 40 vergrößern lässt.

Die Figur 2 zeigt ein Ausführungsbeispiel der Strahlungseinrichtung 40 in einem Behandlungsbetrieb.

Die Strahlungseinrichtung 40 weist eine Strahlungssonde 41 auf, die für die Desinfektion des Behälters 2 in diesen einbringbar ist. Zu diesem Zweck ist die Strahlungssonde 41 bewegbar, insbesondere vertikal verschiebbar, installiert.

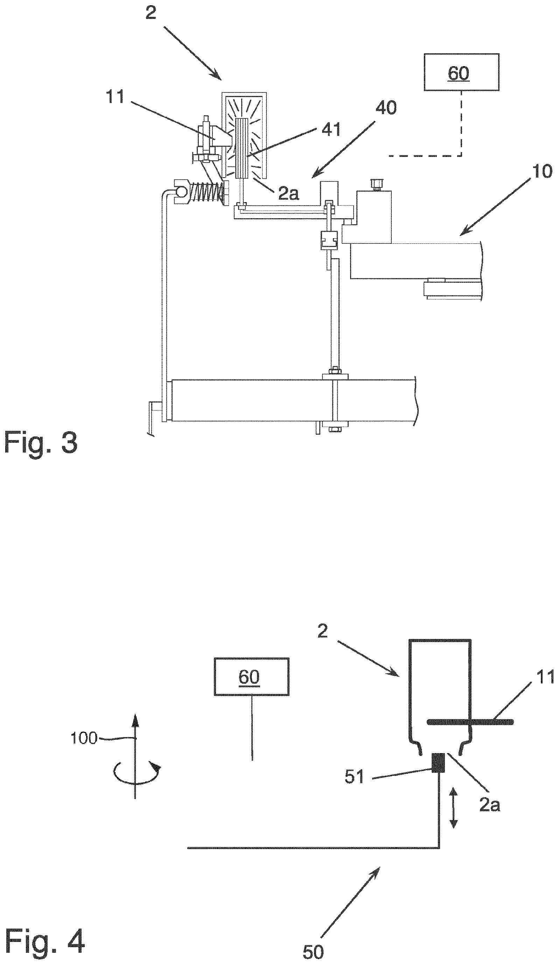

Alternativ oder zusätzlich kann der Behälter 2 so verschiebbar oder verschwenkbar durch die Haltevorrichtung 11 gehalten werden, dass die Strahlungssonde 41 in den Behälter 2 eintauchen kann, wie im Ausführungsbeispiel der Figur 3 gezeigt. Die Haltevorrichtung 11 ist in diesem Fall eingerichtet, um den Behälter 2 aufzuschwenken, so dass die Behältermündung 2a nach unten orientiert ist, und über die Strahlungssonde 41 zu stülpen, so dass diese in den Behälter 2 eindringt.

Indem die Strahlungssonde 41 auf diese Weise in den offenen Behälter 2 eintaucht, können Abschattungen und dadurch Behälterflächen, die durch eine etwaige Abschattung anderweitig nicht oder nur indirekt bestrahlt werden könnten, minimiert werden. Die Desinfektion erfolgt auf diese Weise besonders gründlich und zuverlässig.

Die Behältermündung 2a ist während der Behandlung vorzugsweise nach unten orientiert (in Schwerkraftrichtung gesehen), d.h. der Behälter 2 ist oder wird vorzugsweise auf den Kopf geschwenkt, wodurch etwaige Verunreinigungen, Partikel usw. herausfallen können und sich nicht am Behälterboden sammeln.

Vorzugsweise weist die Vorrichtung 1 zumindest eine Vorbehandlungseinrichtung 50 auf, die eingerichtet ist, um die zu desinfizierenden Behälter 2 vor der Strahlungsbehandlung mit einem Behandlungsmedium auszublasen beziehungsweise auszuspülen.

In der Figur 4 ist eine Vorbehandlungseinrichtung 50 gemäß einem Ausführungsbeispiel, das sich von der Figur 1 unterscheidet, schematisch gezeigt. Mit der Vorbehandlungseinrichtung 50 können die Innenflächen des Behälters 2 mit einem Behandlungsmedium beaufschlagt werden. Eine oder mehrere Behandlungsdüsen 51, fluidtechnisch an eine nicht gezeigte Quelle für das Behandlungsmedium angeschlossen, blasen beziehungsweise spülen das Behandlungsmedium in den Behälter 2. Behälter 2 und/oder Behandlungsdüsen 51 können verschiebbar eingerichtet sein, so dass die Behandlungsdüsen 51 zumindest teilweise in den Behälter 2 eintauchbar sind.

Der Behälter 2 wird bei der Vorbehandlung vorzugsweise mit seiner Mündung 2a nach unten ausgerichtet über die Behandlungsdüse 51 gehalten, wobei aus der Behandlungsdüse 51 das entsprechende Behandlungsmedium in den Innenraum des Behälters 2 eingebracht wird. Das Behandlungsmedium verlässt den Innenraum des Behälters 2 über die Behältermündung 2a. Die Behandlungsdüse 51 schließt in dem gezeigten Ausführungsbeispiel mit der Behältermündung 2a nicht abdichtend ab, sondern es besteht zumindest ein Ringspalt zwischen der Behältermündung 2a und der Behandlungsdüse 51. Alternativ kann die Behältermündung bei der Vorbehandlung geschlossen sein, wobei das Behandlungsmedium in diesem Fall über eine Rückführleitung zurückgeführt und gegebenenfalls für eine weitere Vorbehandlung aufbereitet werden kann.

Die Figur 1 zeigt die Vorbehandlungseinrichtung 50 außerhalb des Behandlungskarussells 10. Die Vorbehandlungseinrichtung 50 beziehungsweise deren Behandlungsdüsen 51 können stationär oder mitlaufend ausgebildet sein. Alternativ kann die Vorbehandlungseinrichtung 50 wie die gezeigte Strahlungseinrichtung 40 Teil des Rundläufers sein. In diesem Fall sind vorzugsweise mehrere Behandlungsdüsen 51, entsprechend den Haltevorrichtungen 11 zugeordnet, am Außenumfang des Behandlungskarussells 10 installiert.

Das Behandlungsmedium ist vorzugsweise Sterilluft, insbesondere ionisierte Sterilluft. In diesem Fall wirkt das Behandlungsmedium im Wesentlichen rein mechanisch, indem etwaige Partikel usw. ausgespült werden. Zusätzlich oder alternativ kann das Behandlungsmedium auch eine chemische Wirkung entfalten, beispielsweise desinfizierend wirken, um die Gesamtbehandlung, umfassend die Vor- und Strahlungsbehandlung, zu optimieren.

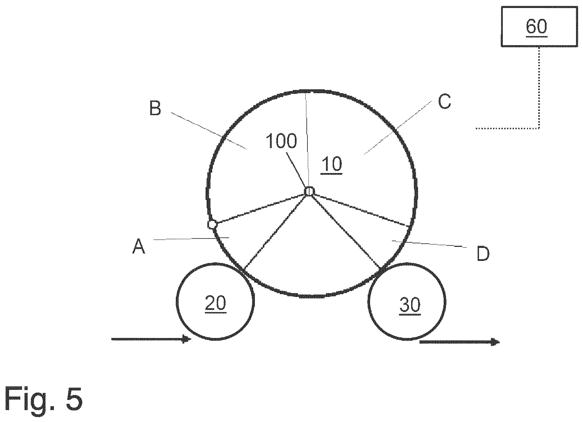

Die Figur 5 zeigt schematisch ein Ausführungsbeispiel der Vorrichtung 1, bei dem das Behandlungskarussell 10 mehrere Behandlungsbereiche A bis D durchfährt. Die Behandlungsbereiche A bis D unterteilen den Förderweg F in vier Sektoren. Im ersten Behandlungsbereich A werden die aufrecht übergebenen Behälter 2 aufgeschwenkt, d.h. durch die Haltevorrichtungen 11 auf den Kopf geschwenkt. Im zweiten Behandlungsbereich B erfolgt die Vorbehandlung der Behälter 2 mittels der Vorbehandlungseinrichtung 50. Im dritten Behandlungsbereich C erfolgt die Desinfektion der Behälter 2 mittels der Strahlungseinrichtung 40. Abschließend werden die Behälter 2 im vierten Behandlungsbereich D vor der Übergabe an den Auslaufstern 30 in die aufrechte Lage ab- beziehungsweise zurückgeschwenkt.

Die Ansteuerung der Transportvorrichtung 10, der Strahlungseinrichtung 40, der Vorbehandlungseinrichtung 50 und etwaiger weiterer Komponenten, wie etwa Sensoren, Aktuatoren usw., erfolgt über eine Steuerung 60.

Die Kommunikation zwischen der Steuerung 60 und den entsprechenden Komponenten kann drahtgebunden oder drahtlos, digital oder analog erfolgen. Die Kommunikation muss nicht notwendigerweise einen Informationsaustausch in beide Richtungen umfassen. Ein unidirektionaler Daten- und/oder Signalfluss fällt hierin unter den Begriff der "Kommunikation". Die Steuerung 60 muss nicht unbedingt durch eine zentrale Recheneinrichtung oder elektronische Regelung gebildet sein, sondern es sind dezentrale und/oder mehrstufige Systeme, Regelungsnetzwerke, Cloud-Systeme und dergleichen umfasst. Die Steuerung 60 kann zudem integraler Bestandteil einer übergeordneten Anlagensteuerung sein oder mit einer solchen kommunizieren.

Die vorstehend beschriebene Vorrichtung 1 ist für die Behandlung von Behältern 2 mit einfacher Geometrie und großer Behältermündung 2a, insbesondere Dosen, besonders geeignet. Die Strahlungssonde 41 und/oder die Behandlungsdüse 51 können wegen der großen Behältermündung 2a tief eintauchen. Ferner gewährleistet eine schlichte, zylindrische Behälterform eine schattenfreie Behandlung.

Soweit anwendbar, können alle einzelnen Merkmale, die in den Ausführungsbeispielen dargestellt sind, miteinander kombiniert und/oder ausgetauscht werden, ohne den Bereich der Erfindung zu verlassen.

### Bezugszeichenliste

- 1: Vorrichtung zum Desinfizieren von Behältern
- 2: Behälter
- 10: Behandlungskarussell
- 11: Haltevorrichtung
- 100: Drehachse
- 20: Einlaufstern
- 30: Auslaufstern
- 40: Strahlungseinrichtung
- 41: Strahlungssonde
- 50: Vorbehandlungseinrichtung
- 51: Behandlungsdüse
- 60: Steuerung

- F: Förderweg

- A: Erster Behandlungsbereich
- B: Zweiter Behandlungsbereich
- C: Dritter Behandlungsbereich
- D: Vierter Behandlungsbereich

## Patentansprüche

1. Vorrichtung (1) zum Desinfizieren eines Behälters (2) mit einer Behältermündung (2a), vorzugsweise in einer Getränkeabfüllanlage, wobei die Vorrichtung (1) aufweist:
eine Transportvorrichtung, die eingerichtet ist, um den Behälter (2) entlang eines Förderwegs (F) zu transportieren;
eine Strahlungseinrichtung (40) mit zumindest einer Strahlungssonde (41), die eingerichtet ist, um eine desinfizierende Strahlung, vorzugsweise UV-Strahlung, zu emittieren, wobei die Strahlungssonde (41) und/oder die Transportvorrichtung so relativ zueinander bewegbar sind, dass die Strahlungssonde (41) durch die Behältermündung (2a) zumindest teilweise in den Behälter (2) einbringbar ist;
eine Steuerung (60), die mit der Transportvorrichtung und der Strahlungseinrichtung (40) in Kommunikation steht und eingerichtet ist, um diese für eine Strahlungsbehandlung zu veranlassen, die Strahlungssonde (41) durch die Behältermündung (2a) in den Behälter (2) einzubringen und mit der desinfizierenden Strahlung zu behandeln.

2. Vorrichtung (1) nach Anspruch 1, **dadurch gekennzeichnet, dass** die Strahlungssonde (41) verschiebbar, vorzugsweise vertikal verschiebbar, ausgebildet ist.

3. Vorrichtung (1) nach Anspruch 1 oder 2, **dadurch gekennzeichnet, dass** die Transportvorrichtung ein um eine Drehachse (100) drehbares Behandlungskarussell (10) umfasst, an dessen Außenumfang mehrere Haltevorrichtungen (11) installiert sind, die eingerichtet sind, um je einen Behälter (2) zu halten und durch Drehung des Behandlungskarussells (10) entlang eines kreisförmigen Förderwegs (F) zu transportieren.

4. Vorrichtung (1) nach Anspruch 3, **dadurch gekennzeichnet, dass** mehrere Strahlungseinrichtungen (40) vorgesehen sind, die am Außenumfang des Behandlungskarussells (10) installiert und je einer Haltevorrichtung (11) zugeordnet sind, so dass mehrere Behälter (2) gleichzeitig behandelbar sind.

5. Vorrichtung (1) nach einem der vorigen Ansprüche, **dadurch gekennzeichnet, dass** die Transportvorrichtung eingerichtet ist, um den Behälter (2) mit der Behältermündung (2a) nach unten orientiert zu transportieren.

6. Vorrichtung (1) nach Anspruch 3 oder 4 und Anspruch 5, **dadurch gekennzeichnet, dass** die Haltevorrichtungen (11) eingerichtet sind, um den entsprechenden Behälter (2) von einer aufrechten Lage, in der die Behältermündung (2a) nach oben orientiert ist, in eine aufgeschwenkte Lage, in der die Behältermündung (2a) nach unten orientiert ist, zu schwenken.

7. Vorrichtung (1) nach einem der vorigen Ansprüche, **dadurch gekennzeichnet, dass** die Strahlungseinrichtung (40) eingerichtet ist, um über die Strahlungssonde (41) eine gepulste Strahlung, vorzugsweise mit einer Pulsdauer im Bereich von 0,2 ms bis 0,4 ms, zu emittieren, wobei die Wellenlänge der Strahlung vorzugsweise im Bereich von 300 nm bis 400 nm liegt.

8. Vorrichtung (1) nach einem der vorigen Ansprüche, **dadurch gekennzeichnet, dass** diese ferner eine Vorbehandlungseinrichtung (50) mit zumindest einer Behandlungsdüse (51) aufweist, die eingerichtet ist, um den Behälter (2) mit einem Behandlungsmedium, vorzugsweise Sterilluft, auszuspülen, wobei die Steuerung (60) mit der Vorbehandlungseinrichtung (50) in Kommunikation steht und eingerichtet ist, um diese für eine Vorbehandlung zu veranlassen, den Behälter (2) vor der Strahlungsbehandlung durch die Strahlungseinrichtung (40) mit dem Behandlungsmedium auszuspülen.

9. Vorrichtung (1) nach Anspruch 8, **dadurch gekennzeichnet, dass** die Behandlungsdüse (51) durch die Behältermündung (2a) zumindest teilweise in den Behälter (2) einbringbar ist, vorzugsweise vertikal verschiebbar ist.

10. Vorrichtung (1) nach einem der vorstehenden Ansprüche, **dadurch gekennzeichnet, dass** diese zum Desinfizieren eines oder mehrerer Behälter (2) in der Form im Wesentlichen eines senkrechten Kreiszylinders, vorzugsweise von Dosen, eingerichtet ist.

11. Verfahren zum Desinfizieren eines Behälters (2) mit einer Behältermündung (2a), vorzugsweise in einer Getränkeabfüllanlage, wobei das Verfahren aufweist:
Transportieren des Behälters (2) mittels einer Transportvorrichtung entlang eines Förderwegs (F), wobei der Behälter (2) vorzugsweise mit der Behältermündung (2a) nach unten orientiert transportiert wird;
Einbringen einer Strahlungssonde (41) einer Strahlungseinrichtung (40) durch die Behältermündung (2a) in den Behälter (2); und
Durchführen einer Strahlungsbehandlung durch Emittieren einer desinfizierenden Strahlung, vorzugsweise UV-Strahlung, durch die Strahlungssonde (41), um den Behälter (2) mit der desinfizierenden Strahlung zu behandeln.

12. Verfahren nach Anspruch 11, **dadurch gekennzeichnet, dass** die desinfizierende Strahlung eine gepulste Strahlung, vorzugsweise mit einer Pulsdauer im Bereich von 0,2 ms bis 0,4 ms, ist, wobei die Wellenlänge der Strahlung vorzugsweise im Bereich von 300 nm bis 400 nm liegt.

13. Verfahren nach Anspruch 11 oder 12, **dadurch gekennzeichnet, dass** vor der Strahlungsbehandlung eine Vorbehandlung mittels einer Vorbehandlungseinrichtung (50) durchgeführt wird, indem der Behälter (2) mit einem Behandlungsmedium, vorzugsweise Sterilluft, ausgespült wird.

14. Verfahren nach einem der Ansprüche 11 bis 13, **dadurch gekennzeichnet, dass** der Behälter (2) im Wesentlichen die Form eines senkrechten Kreiszylinders aufweist, vorzugsweise eine Dose ist.

15. Verfahren nach Anspruch 11 und 13, **dadurch gekennzeichnet, dass** die Transportvorrichtung ein um eine Drehachse (100) drehbares Behandlungskarussell (10) umfasst, an dessen Außenumfang mehrere Haltevorrichtungen (11) installiert sind, die eingerichtet sind, um je einen Behälter (2) zu halten und durch Drehung des Behandlungskarussells (10) entlang eines kreisförmigen Förderwegs (F) zu transportieren und zu schwenken, wobei das Behandlungskarussell (10) mehrere Behandlungsbereiche (A bis D) durchfährt, umfassend
einen ersten Behandlungsbereich (A), in dem der Behälter (2) durch die entsprechende Haltevorrichtung (11) von einer aufrechten Lage, in der die Behältermündung (2a) nach oben orientiert ist, in eine aufgeschwenkte Lage, in der die Behältermündung (2a) nach unten orientiert ist, geschwenkt wird;
einen zweiten Behandlungsbereich (B), in dem die Vorbehandlung des Behälters (2) mittels der Vorbehandlungseinrichtung (50) durchgeführt wird;
einen dritten Behandlungsbereich (C), in dem die Strahlungsbehandlung des Behälters (2) mittels der Strahlungseinrichtung (40) durchgeführt wird; und
einen vierten Behandlungsbereich (D), in dem der Behälter (2) durch die entsprechende Haltevorrichtung (11) in die aufrechte Lage, in der die Behältermündung (2a) nach oben orientiert ist, zurückgeschwenkt wird.
